# EUROPEAN PATENT APPLICATION

(11) **EP 3 904 512 A1**
(43) Date of publication of application: **03.11.2021**
(21) Application number: 19905395.0
(22) Date of filing: 14.01.2019
(51) Int. Cl.: C12N 15/10, C12Q 1/6806

(54) **METHOD FOR QUICKLY EXTRACTING LONG-FRAGMENT GENOMIC DNA BY SINGLE REACTION TUBE, AND KIT**

(30) Priority: 27.12.2018 CN 201811619310
(71) Applicant: Berry Genomics Co., Ltd., Beijing 102206 (CN)
(72) Inventor: MAO, Aiping, Beijing 102206 (CN); ZHANG, Haiman, Beijing 102206 (CN); ZHANG, Jianguang, Beijing 102206 (CN)
(74) Representative: Plasseraud IP
(86) International application number: PCT/CN2019/071678
(87) International publication number: WO 2020/133600

(57) **Abstract**

A method for quickly extracting long-fragment genomic DNAs by a single reaction tube, comprising the following steps: a) adding a lysis solution and protease K to a sample, to lyse cells and release the genomic DNAs; b) adding a precipitant to the reaction system of step a) to obtain a precipitate of the genomic DNAs; c) washing the obtained precipitate of the genomic DNAs using a washing solution; and d) dissolving the genomic DNAs using a dissolution solution. Also provided is a kit suitable for the method above.

## Description

### FIELD OF THE INVENTION

The present invention relates to the field of extracting genomic DNAs. In particular, the present invention relates to a method for quickly extracting long-fragment genomic DNAs by a single reaction tube, and a kit suitable for the method.

### BACKGROUND

The preparation of long-fragment genomic DNA molecules has a wide demand in the field of biology, particularly DNA sequencing and genome assembly. The SMRT long-read long-sequencing technology of Pacific Biosicences may achieve 60-100 Kb in length for single-molecule sequencing ¹, while the nanopore sequencing technology of Oxford Nanopore Technology is able to detect single-molecule DNA fragments up to Mb scale in length ². Linked-reads technologies such as 10 x Genomics³ and CPT-seq⁴ for haplotype splicing and genome assembly require the preparation of genomic molecular fragments of tens to hundreds of Kb in length. The single-molecule optical mapping technology based on the Saphyr platform of Bionano Genomics can assist in genome assembly, detection of chromosomal structural variation and genetic diseases. The molecular length of its marker N50 (≥ 150 Kb) is about 300 Kb, and the longest single molecule is up to Mb scale⁵. With the development of sequencing and optical mapping technologies, there is a higher demand for simple and rapid extraction of the long-fragment genomic DNA molecules.

At present, the main methods for extracting the genomic DNAs are phenol chloroform extraction-ethanol/isopropanol precipitation, silica gel membrane column or adsorption magnetic bead purification. Due to mechanical and shear forces, the length of the genomic DNAs obtained by conventional silica membrane column or adsorption magnetic bead purification is less than 50 Kb. Moreover, the classical method of phenol chloroform extraction-ethanol/isopropanol precipitation requires using the organic solvent phenol chloroform, which is highly toxic. Moreover, due to the high viscosity after cell lysis, the operation of extraction in the liquid phase of phenol chloroform is quite difficult ⁶. The phenol chloroform extraction and isolation issues may be solved by using Phase-lock gel, while the whole process is still very cumbersome ². In recent years, some novel methods for extracting long-fragment genomic DNAs based on silicon wafers or nucleic acid probes have been reported, which however have not been widely used due to the limitations of materials or procedures ^{7, 8}. In addition, the above method can be used to extract the genomic DNA fragments up to hundreds Kb in length, but still fails to reach Mb scale.

Currently, the most classical method for extracting high-purity long-fragment genomic DNAs is solid-phase genomic DNA extraction based on low-melting point agarose gel-embedding, which can be used for the extraction of high-purity genomic DNAs up to Mb scale in length. However, it comprises steps of embedding cells into agarose gel, the treatment of proteinase K digestion, the lysis and enzymolysis of gel, and the dialysis of genomic DNAs, and the like, and the operation process is quite tedious and takes more than 24 hours, not suitable for large-scale applications. Furthermore, the above-mentioned methods for extracting genome involve the transfer of the genomic DNAs in different reaction tubes, which is not only cumbersome in operation, but also leads to the loss of the genomic DNAs and even the contamination of the genomic DNAs.

In view of the above, the present invention provides a novel method for extracting long-fragment genomic DNAs, which comprises only four steps of lysing cells or tissues, precipitating, washing and dissolving the genomic DNAs. The method may rapidly extract the genomic DNAs of Mb scale in length; significantly shorten the time spent for extraction of the genomic DNAs, and allow the extraction process more rapid and simple. In addition, the process of the present invention can be carried out in a single reaction tube without a tube-transfer, saving time and avoiding contamination or loss of the genomic DNAs. Finally, the precipitated long-fragment genomic DNAs are easy to dissolve with high homogeneity, fulfilling the requirements of various long-fragments sequencing and optical mapping technologies.

### CONTENT OF THE INVENTION

The purpose of the present invention is to solve the problems of the existing long-fragment genomic DNA extraction, which is time-consuming and complicated to operate. The present invention achieves the goal of rapidly preparing the long-fragment genomic DNAs by directly lysing cells, releasing, precipitating, washing and dissolving the genomic DNAs in a single reaction tube.

Accordingly, in a first aspect, the present invention provides a method for extracting long-fragment genomic DNAs comprising the following steps:
a) adding a lysis solution and proteinase K to a sample, to lyse cells and release the genomic DNAs;
b) adding a precipitant to the reaction system of step a) to obtain a precipitate of the genomic DNAs;
c) washing the resulting precipitate of the genomic DNAs using a washing solution;
d) dissolving the genomic DNAs using a dissolution solution.

In one embodiment, the method of the present invention consists of the above four steps a)-d).

As used herein, "long-fragment genomic DNA" refers to genomic DNAs having an average length greater than 200 Kb and up to Mb scale.

In one embodiment, the sample can be any tissue or cell of animal origin, e.g. cells from embryonic tissue, liver tissue, thymus tissue, spleen tissue, and body fluid, or cell lines cultured *in vitro,* and the like. Examples of body fluids include, but not limited to, blood, serum, plasma, synovial fluid, semen, urine, sweat, saliva, feces, cerebrospinal fluid, ascites, pleural fluid, bile, pancreatic fluid, and the like.

In one embodiment, the lysis solution comprises NaCl, Tris-HCl (pH 7.4-8.0), EDTA (pH 8.0), and SDS. In another embodiment, the lysis solution consists of NaCl, Tris-HCl (pH 7.4-8.0), EDTA (pH 8.0) and SDS. The concentration of NaCl, Tris-HCl (pH 7.4-8.0) and EDTA (pH 8.0) in the lysis solution can be adjusted and determined by conventional experiments according to specific experimental requirements, for example, the concentration of NaCl is 0.1m-0.5m, of Tris-HCl (pH 7.4-8.0) is 10 mM-200 mM and of EDTA (pH 8.0) is 10 mM-100 mM. However, the concentration of SDS is critical to the quality of the extracted genomic DNAs. Specifically, when the concentration of SDS is too low, e.g. less than 0.1%, insufficient cell lysis may occur; while the concentration of SDS is too high, e.g. above 8%, the excess SDS will affect the subsequent experiments as the genomic DNA precipitates. Therefore, in the present invention, the concentration of SDS is preferably from 0.1% to 8%, more preferably from 0.1% to 5%.

In one embodiment, the lysis solution may further comprise RNAse A, which functions to remove RNA impurities in the system. The amount of RNAse A can be determined by one skilled in the art as needed, for example, 20 µg/ml.

In one embodiment, the final concentration of proteinase K is 0.005-4 mg/ml, preferably 0.005-2.5 mg/ml. Protease K functions to degrade membrane proteins and proteins that bind to the genomic DNAs, leaving the genomic DNAs sufficiently free. When the content of proteinase K is too low, for example, less than 0.005 mg/ml, the membrane proteins and proteins bound to the genomic DNAs may not be fully degraded, which may affect the subsequent application of the genomic DNAs, e.g., the BioNano optical mapping labeling of the genomic DNAs; while the content of proteinase K is too high, e.g. above 4 mg/ml, with the precipitation of the genome DNAs, the excess proteinase K may degrade the cleavage system in subsequent applications, e.g. the BioNano optical mapping labeling of the genomic DNAs.

In one embodiment, proteinase K and the lysis solution may be added sequentially or simultaneously.

The time and temperature of the treatment in the above step a) can be adjusted and determined by one skilled in the art according to experimental requirements. For example, the treatment may be carried out at a temperature of 30 to 65°C for 1 to 2 hours.

In one embodiment, the precipitant of the present invention comprises at least one inorganic salt and at least one alcohol. In another embodiment, the precipitant of the present invention consists of inorganic salts and alcohols. In the method of the present invention, examples of inorganic salts include, but not limited to, ammonium acetate, sodium acetate, sodium chloride, lithium chloride, and the like; examples of alcohols include, but not limited to, anhydrous ethanol, isopropanol, and the like. The concentration of inorganic salts suitable for precipitating the genomic DNAs in the invention is 300-500 mM, preferably 350-450 mM, more preferably about 450 mM, and the concentration of alcohols is 50-80%, more preferably 60-70%, further more preferably about 65%.

The precipitation time in the above step b) can be adjusted and determined by one skilled in the art depending on experimental requirements.

In one embodiment, the washing solution comprises 70% to 80% ethanol. In another embodiment, the washing solution consists of 70% to 80% ethanol.

In one embodiment, the dissolution solution is selected from deionized water, Tris-HCl, TE buffer, and the like. Other known solutions that can be used to dissolve the genomic DNAs are also suitable for use in the present invention.

In a second aspect, the invention provides a kit for extracting the long-fragment genomic DNAs comprising: lysis solution, proteinase K, optionally RNAse A, precipitant, washing solution and dissolution solution.

In one embodiment, the lysis solution comprises NaCl, Tris-HCl (pH 7.4-8.0), EDTA (pH 8.0), and SDS. In another embodiment, the lysis solution consists of NaCl, Tris-HCl (pH 7.4-8.0), EDTA (pH 8.0) and SDS. The concentration of NaCl, Tris-HC (pH 7.4-8.0) and EDTA (pH 8.0) in the lysis solution can be adjusted and determined by conventional experiments according to specific experimental requirements, for example, the concentration of NaCl is 0.1m-0.5m, of Tris-HCl (pH 7.4-8.0) is 10 mM-200 mM and of EDTA (pH 8.0) is 10 mM-100 mM. In the present invention, the concentration of SDS is preferably 0.1% to 8%, more preferably 0.1% to 5%.

Preferably, the concentration of proteinase K is from 0.005 to 4 mg/ml, more preferably from 0.005 to 2.5 mg/ml. When RNAse A is present, the amount thereof is 20 µg/ml.

In one embodiment, the precipitant comprises at least one inorganic salt and at least one alcohol. In another embodiment, the precipitant of the present invention consists of inorganic salts and alcohols. In the process of the present invention, examples of inorganic salts include, but not limited to, ammonium acetate, sodium acetate, sodium chloride and the like; examples of alcohols include, but not limited to, anhydrous ethanol, isopropanol, and the like. The concentration of inorganic salts suitable for precipitating the genomic DNAs in the present invention is 300-500 mM, preferably 350-450 mM, more preferably about 450 mM. The concentration of alcohols is 50-80%, preferably 60-70%, more preferably about 65%.

In one embodiment, the washing solution comprises 70% to 80% ethanol. In another embodiment, the washing solution consists of 70% to 80% ethanol.

In one embodiment, the dissolution solution is selected from deionized water, Tris-HCl, TE buffer, and the like.

The method and kit of the present invention allow a rapid, simple and accurate extraction of the long-fragment genomic DNAs in a single reaction tube. The excellent technical effects of the method and kit of the present invention are mainly embodied in the following aspects:
(1) All the operations are carried out in a single reaction tube, without the transfer of the genomic DNAs within different reaction tubes involved, thus saving time and costs while avoiding loss and potential contamination of the genomic DNAs.
(2) The whole experimental procedure is simple and rapid. The whole genomic DNA extraction process contains only four steps of lysis, precipitation, washing and dissolution, and takes 1-2 hours.
(3) The extraction method of the present invention is relatively safe and does not pose a risk to the health of the operator. The reagents used in the extraction methods of the present invention are safe and non-toxic, while further avoiding the use of highly toxic organic solvents such as phenol chloroform that are used in traditional precipitation of the genomic DNAs.
(4) The length and quality of the extracted genomic DNAs are reliable and the method is highly reproducible. The present invention avoids mechanical damage to the genomic DNAs compared to conventional methods of silica gel membrane columns and magnetic bead purification. The present invention provides significant improvements in experimental operability and reproducibility over classical low-melting point agarose gel embedding-based methods for extracting the long-fragment genomic DNAs.

The method for extracting the genomic DNAs of the present invention is suitable for multiple long-fragment sequencing and optical mapping technology platforms.

The invention will be further illustrated with reference to the drawings and examples. It should be understood that the following examples are illustrative only and not intended to limit the scope of the present invention.

### BRIEF DESCRIPTION OF THE DRAWINGS

Figure 1: Pulsed field electropherogram of the genomic DNAs in Example 3. M: marker; 1: the genomic DNAs extracted in example 2; 2: the genomic DNAs extracted in example 1; and the white arrow shows a DNA fragment of about 2200 kb in length in lane 2.
Figure 2: Results of DLS labeling of the long-fragment genomic DNAs extracted with different concentrations of proteinase K.
Figure 3: Results of DLS labeling of the long-fragment genomic DNAs extracted with different concentrations of SDS.

### DETAILED DESCRIPTION

### Example 1. Extraction of long-fragment genomic DNAs according to the method of the present invention

The genomic DNAs of leukocytes were extracted by using the method of the invention according to the following steps:
(1) 0.3x10⁶ leukocytes were centrifuged at 2000g for 2 min at room temperature to remove the supernatant;
(2) 30 µL lysis solution (100 mM NaCl, 10 mM Tris-HCl (pH 8.0), 25 mM EDTA (pH 8.0) and 0.50% SDS) was added to resuspend the cells, and then 2 µL of 2 mg/ml proteinase K was added and mixed well;
(3) The mixture was placed in a metal bath and treated at 50°C for 1 hour;
(4) 12 µL of 5M ammonium acetate and 90 µL of anhydrous ethanol were added, inverted up and down for 10 times, and then placed on a shaker to slowly mix well for 5-10 min at room temperature and 20 rpm, so as to completely precipitate the DNAs and form a small mass;
(5) The DNAs were washed twice with 200 µL of 70% ethanol;
(6) The DNAs were dissolved with 42 µL of TE buffer (10 mmol/L Tris-HCl (pH 8.0); 1 Mmol/L EDTA (pH 8.0)) to obtain the long-fragment genomic DNAs.

### Example 2. Extraction of long-fragment genomic DNAs according to the method of low melting point agarose embedding

The genomic DNAs were extracted from leukocytes using a conventional method of low melting point agarose embedding according to the following steps:
Step 1: preparation of agarose gel blocks
   (1) 0.3 x10⁶ leukocytes were centrifuged at 2000 g for 2 min to remove supernatant.
   (2) The cells were resuspended with 65 µL of PBS and equilibrateD in a 43°C metal bath for 10 min.
   (3) 39 µL of 2% agarose was added for mixing, and the mixture was added to gel holes.
   (4) The gel was placed to form blocks in a refrigerator at 4°C for 45 minutes.
Step 2: digestion of gel block
   (5) 2.5 mL of proteinase K solution was added to a 50 mL centrifuge tube. The gel blocks were transferred into the 50 mL centrifuge tube, and placed in a thermostatic mixer for mixing at 50°C for 2 hours.
   (6) A sieve cover was added, then the proteinase K solution was poured out and replaced with fresh proteinase K solution; and placed in a thermostatic mixer at 50°C and mixed well overnight.
Step 3: washing of the gel block
   (7) A mesh cap was added and the overnight proteinase K solution was poured off. 10mL of washing buffer (10 mM Tris-HCl, 50 mM EDTA) was added, then placed on a horizontal shaker for slow shaking for 5 minutes, a mesh cap was added and the washing buffer waspoured off. The process was repeated for a total of 4 washes.
   (8) The gel block was washed for 5 times with 1 x TE buffer replaced, as above.
Step 4: melting of the gel block
   (9) The gel block was removed with a little spoon, the water was sucked up with KimWipe and then the gel block was transferred to a 1.5 mL centrifuge tube; the gel block was instantaneously released to the tube bottom, and the residual liquid was sucked away using a small pipette tip.
   (10) The gel block was treated in a metal bath at 70°C for 2 minutes to melt the gel.
   (11) The tube was rapidly transferred in 43°C metal bath for 5 minutes, then 2 µL of Agarose was added, and placed in 43°C metal bath for additional 45 minutes.
   (12) 0.1 µm filter membrane was placed in a 6 cm culture dish containing 10-15 mL of 1 x TE buffer, to keep the membrane float on the liquid surface. The sample was added on the filter membrane and dialyzed for 45 minutes.
   (13) The dialyzed sample was pipetted into a 1.5 mL centrifuge tube and gently blown till homogeneous with a pipette tip to obtain the long-fragment genomic DNAs.

### Example 3. Quality detection of long-fragment genomic DNAs

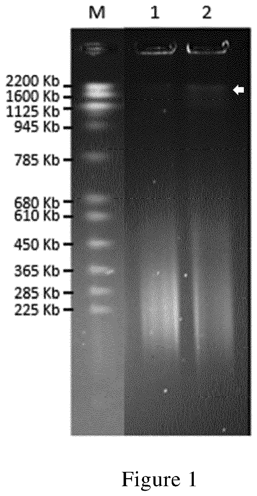

The long-fragment genomic DNAs obtained in Examples 1 and 2 were subjected to pulsed field electrophoresis to examine its quality, and the results are shown in Fig. 1, indicating that the long-fragment DNAs with an average length of more than 200 kb and up to the Mb scale can be efficiently obtained by the method of the present invention.

### Example 4. Detection of the effect of the lysis with different concentrations of proteinase K and SDS on the quality of extracted long-fragment genomic DNAs

In order to detect the effect of the lysis with different concentrations of proteinase K and SDS on the quality of the extracted long-fragment genomic DNAs, the long-fragment genomic DNAs of leukocytes were extracted using different concentrations of proteinase K and SDS according to the method described in Example 1. The extracted genomic DNAs were then labeled using the BioNano Genomics Company's DLS DNA labeling kit according to the manufacturer's instructions and data collection was performed on the Saphyr platform of BioNano Genomics Company.

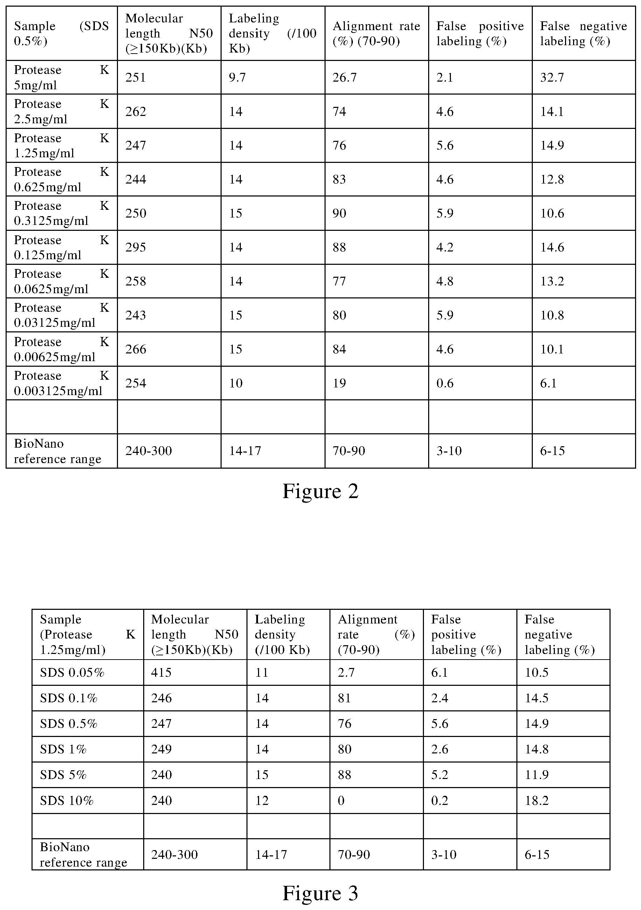

As shown in Fig.2, when the concentration of proteinase K is too high, e.g. 5 mg/ml, the labeling density and the alignment rate of the extracted long-fragment DNAs are extremely low as well as the false negative labeling ratio is too high to meet the requirement of the ideal labeling (determined according to the reference range). However, when the proteinase K concentration is too low, e.g. 0.003125 mg/ml, the labeling density and the alignment rate of the extracted long-fragment DNAs are still too low to meet the requirement of the ideal labeling.

As shown in Figure 3, when the SDS concentration is too high, e.g. 10%, the labeling density and the alignment rate of the extracted long-fragment DNAs are both extremely low as well as the false negative labeling ratio is too high to meet the requirement of the ideal labeling (determined according to the reference range). However, when the SDS concentration is too low, e.g. 0.05%, the labeling density and the alignment rate of the extracted long-fragment DNAs are still too low, and the length of the molecule N50 is also far beyond the reference range, and not meeting the requirement of the ideal labeling.

Thus, in order for the quality of the extracted long-fragment DNAs to be high enough to meet the requirements for the subsequent direct sequencing and assembly (e.g. in the case of the single-molecule optical mapping technique of BioNano, the quality of the long-fragment DNAs should be high enough to achieve the ideal labeling requirements before genome assembly can be performed effectively without affecting its completeness and accuracy), and the concentration of proteinase K and SDS should be controlled within a certain range when extracting the long-fragment DNAs. For example, the protease concentration is preferably from 0.005 to 4 mg/ml, more preferably from 0.005 to 2.5 mg/ml; and the SDS concentration is preferably 0.1-8%, more preferably 0.1-5%.

It should be noted that while some of the features of the present disclosure have been illustrated by the above examples, they are not intended to limit the invention and that the method for quickly extracting the long-fragment genomic DNAs by single-reaction tube and kit set forth herein may have a variety of application scenarios. It will be apparent to those skilled in the art that various modifications and variations can be made in the present invention. It will thus be appreciated that those skilled in the art will be able to devise various substitutions which should be included within the scope of the disclosure without departing from the concept and principle of the invention.

### REFERENCE

[1] Roberts RJ, et al. The advantages of SMRT sequencing. Genome Biol. 2013 Jul 3;14(7):405. Erratum in: Genome Biol. 2017 Aug 16;18(1):156.
[2] Jain M, et al. Nanopore sequencing and assembly of a human genome with ultra-long reads. Nat Biotechnol. 2018 Apr;36(4):338-345.
[3] Zheng GX, et al. Haplotyping germline and cancer genomes with high-throughput linked-read sequencing. Nat Biotechnol. 2016 Mar; 34(3): 303-11.
[4] Zhang F, et al. Haplotype phasing of whole human genomes using bead-based barcode partitioning in a single tube. Nat Biotechnol. 2017 Sep; 35(9):852-857.
[5] Lam ET, et al. Genome mapping on nanochannel arrays for structural variation analysis and sequence assembly. Nat Biotechnol. 2012 Aug; 30(8): 771-6.
[6] Goldenberger D , et al. A simple "universal" DNA extraction procedure using SDS and proteinase K is compatible with direct PCR amplification. PCR Methods Appl. 1995 Jun;4(6):368-70.
[7] Zhang Y, et al. A Simple Thermoplastic Substrate Containing Hierarchical Silica Lamellae for High-Molecular-Weight DNA Extraction. Adv Mater. 2016 Dec; 28(48):10630-10636.
[8] Murphy NM, et al. High Molecular Weight DNA Enrichment with Peptide Nucleic Acid Probes. Methods Mol Biol. 2017; 1551:73-85.

## Claims

1. A method of extracting long-fragment genomic DNAs comprising the following steps:
a) adding a lysis solution and proteinase K to a sample, to lyse cells and release the genomic DNAs;
b) adding a precipitant to the reaction system of step a) to obtain a precipitate of the genomic DNAs;
c) washing the resulting precipitate of the genomic DNAs using a washing solution;
d) dissolving the genomic DNA using a dissolution solution.

2. The method according to claim 1, wherein the sample is tissues or cells of animal origin.

3. The method according to claim 1, wherein the sample is cells from embryonic tissue, liver tissue, thymus tissue, spleen tissue or body fluid, or a cell line cultured *in vitro.*

4. The method according to claim 3, wherein the body fluid is selected from the group consisting of blood, serum, plasma, synovial fluid, semen, urine, sweat, saliva, feces, cerebrospinal fluid, ascites, pleural fluid, bile, and pancreatic fluid.

5. The method according to claim 1, wherein the lysis solution comprises NaCl, Tris-HCl, EDTA, and SDS.

6. The method according to claim 5, wherein the concentration of SDS is from 0.1% to 8%.

7. The method according to claim 5, wherein the lysis solution comprises RNAse A.

8. The method according to claim 1, wherein the concentration of the proteinase K is from 0.005 to 4 mg/ml.

9. The method according to claim 1, wherein the proteinase K and the lysis solution are added sequentially or simultaneously.

10. The method according to claim 1, wherein the precipitant comprises at least one inorganic salt and at least one alcohol.

11. The method according to claim 10, wherein the inorganic salt is selected from the group consisting of ammonium acetate, sodium acetate, and sodium chloride.

12. The method according to claim 10, wherein the alcohol is selected from the group consisting of anhydrous ethanol and isopropanol.

13. The method according to claim 1, wherein the wash solution comprises 70% ethanol.

14. The method according to claim 1, wherein the dissolving solution is selected from the group consisting of deionized water, Tris-HCl, and TE buffer.

15. The method according to claim 1, wherein the method is carried out in a single reaction tube.

16. The method according to claim 1, wherein the long-fragment genomic DNAs are up to Mb scale in length.

17. A kit for extracting long-fragment genomic DNAs comprising: lysis solution, proteinase K, optionally RNAse A, precipitant, washing solution and dissolution solution.

18. The kit according to claim 17, wherein the lysis solution comprises NaCl, Tris-HCl, EDTA, and SDS.

19. The kit according to claim 18, wherein the concentration of SDS is from 0.1% to 8%.

20. The kit according to claim 17, wherein the lysis solution comprises RNAse A.

21. The kit according to claim 17, wherein the concentration of proteinase K is from 0.005 to 4 mg/ml.

22. The kit according to claim 17, wherein the precipitant comprises at least one inorganic salt and at least one alcohol.

23. The kit according to claim 22, wherein the inorganic salt is selected from the group consisting of ammonium acetate, sodium acetate and sodium chloride.

24. The kit according to claim 22, wherein the alcohol is selected from the group consisting of anhydrous ethanol and isopropanol.

25. The kit according to claim 17, wherein the wash solution comprises 70% ethanol.

26. The kit according to claim 17, wherein the dissolution solution is selected from the group consisting of deionized water, Tris-HCl, and TE buffer.
